(19) Europäisches Patentamt
European Patent Office
Office européen des brevets

(11) Publication number: 0 291 232
A1

# (12) EUROPEAN PATENT APPLICATION

(21) Application number: 88304077.6

(22) Date of filing: 05.05.88

(51) Int. Cl.⁴: C12N 11/08 , C12N 11/12 , C12N 5/00 , C12N 5/02 , C12M 1/40 , C12M 3/00

(30) Priority: 15.05.87 CA 537315

(43) Date of publication of application:
17.11.88 Bulletin 88/46

(84) Designated Contracting States:
BE CH DE FR GB IT LI NL SE

(71) Applicant: Canadian Patents and
Development Limited Société Canadienne
des Brevets et d'Exploitation Limitée
275 Slater Street
Ottawa Ontario, K1A 0R3(CA)

(72) Inventor: Archambault, Jean
207 Braebrook Street
Pointe Claire Quebec, H9R 1V6(CA)
Inventor: Volesky, Bohumil
471 Berkley Avenue
St. Laurent Quebec, J4P 3E7(CA)
Inventor: Kurz, Wolfgang G.W.
514 Copland Crescent
Saskatoon Saskatchewan, S7H 2Z5(CA)

(74) Representative: Lambert, Hugh Richmond et
al
D. YOUNG & CO. 10 Staple Inn
London, WC1V 7RD(GB)

(54) Surface immobilization of plant cells.

(57) A novel technique is disclosed for the immobilization of plant cells on a support. The cells are deposited on a surface of fibrous support which provides for strong binding of plant tissue biomass growing in the submerged culture. Specifically the plant cells are immobilised onto the surface of a fibrous support comprising a multiplicity of loosely bound polyester and/or cellulosic fibres by simple contact of the support with a suspension of the plant cells in a culture medium.

EP 0 291 232 A1

## Surface Immobilization of Plant Cells

This invention relates to the immobilization of plant cells on a surface.

Immobilization represents an interesting alternative to the suspension culturing of plant cells for the production of secondary metabolites. The advantages of this technology have been repeatedly emphasized and include:

1 - Promotion of the natural tissue forming tendency of plant cells resulting in biomass homogeneity and morphological rest. (M.E. Curtin, Biotechnology 1, 649 (1983); and M.L. Shuler, J.W. Pyne, and G.A. Hallsby, J. Amer. Oil Chem. Soc. 61, 1724 (1984).

2 - Protection of the fragile plant cells against hydrodynamic stress. (Curtin op. cit; Shuler et al op. cit).

3 - Separation of the biomass from the medium with improved control over hydrodynamics and mass transfer in the culture system. (Shuler et al. op. cit.; O. Sahai and M. Knuth, Biotechol. Prog. 1,1 (1985); and A. Rosevear and C.A. Lambe, in Topics in Enzyme and Fermentation Technology, A. Wiseman ed. Horwood, West Sussex, U.K., 1983, Vol. 7, p. 3);

4 - Improved volumetric efficiency of the culture system. (Curtin op. cit; Shuler et al op. cit; Sahai and Knuth op cit; Rosevear and Lambe op. cit; and P. Brodelius, in Immobilized Cells and Organelles, B. Mattiasson ed., CRC Press, Boca Raton, 1983, Vol. 1, p. 7).

However, this approach is not without problems of which growth restriction (Brodelius 1983 op. cit.; and P. Brodelius, An. N.Y. Acad. Sci. 434, 382 (1982) and product secretion in the medium (Curtin op. cit; Rosevear and Lambe op cit) are crucial to its success.

The techniques developed so far to immobilize plant cells involve mainly biomass entrapment. Crosslinking agent toxicity (glutaraldehyde) (P. Brodelius, and K. Mosbach, J. Chem. Technol. Biotechnol. 32, 330 (1982)) or chelation by medium ions (calcium and phosphate) (P. Brodelius and K. Nilsson, FEBS Lett. 122, 312 (1980)), limited mechanical stability, (Brodelius 1983 op. cit; P. Brodelius and K. Mosbach, Ad. App. Microbiol. 28, 1 (1982); and P. Brodelius, An. N.Y. Acad. Sci. 413, 383 (1983)), and biomass loading values (P. Morris, N.J. Smart, and M.W. Fowler, Plant Cell Tissue Organ Culture 2, 207 (1983)) and inherent difficulties in application restrict the usefulness of the gel entrapment techniques. Hollow fiber cartridges also studied for plant cell immobilization are expensive and require a medium recirculation system which is prone to contamination (W. Jose, H. Pedersen and C. Chin, An. N.Y. Acad. Sci. 413, 409 (1983)).

Polyurethane foam particle entrapment has been described by K. Lindsey, M.M. Yeoman, G.M. Black and F. Mavituna (FEBS lett. 153, 143 (1983)) and F. Mavituna and J.M. Park (Biotechnol. Lett. 7, 637 (1985)). Using this support the plant cell aggregates become physically entrapped in the continuous open pore network and then the aggregates grow and/or adhere to each other to fill the rest of the available space within the foam. This entrapment method has the following disadvantages: the particle is large, which may cause mass transfer and viability problems and the free particulate nature of the system causes difficulties in scale up (K. Lindsey and M.M. Yeoman, Planta 162, 495 (1984); and F. Mavituna, J.M. Park, A.K. Wilkinson, and P.D. Williams, VI International Congress on Plant Tissue and Cell Culture, Univ. of Minnesota, Aug. 1986, Poster #229)).

U.S. Patents 4,378,435 and 4,605,622 both teach methods of immobilizing enzymes or microorganisms onto solid supports. In both of these processes the enzymes or microorganisms are chemically bound to the supports. The chemicals used would be toxic to plant cells and therefore the methods taught in these two patents would not be appropriate for immobilizing plant cells.

We desire to develop a surface immobilization technique based only on natural adhesion which makes it a gentle and easy method particularly suitable for plant cells. Mass transfer is only a function of the support configuration and of the medium flow hydrodynamics, two variables easy to manipulate. Occurrence of loose plant cell biomass in the medium, the most obvious potential problem of this technique reflecting the efficiency of the cell retainment, can be controlled by selecting a proper support material and an appropriate degree of mixing.

The surface immobilization of plant cells as described herein presents certain advantages over the existing immobilization technology by (gel) entrapment such as:

1 - The technique is simple, easy and not stressful to the fragile plant cells. It is carried out under the normal growth conditions used for plant cells;

2 - All the inoculum becomes readily and strongly attached. The growing biomass remains always attached without leakage;

3 - No special chemical additives are required to improve the attachment;

4 - The cell support material is sterilizable with the medium and can easily be formed into different structures;

5 - Foaming and frothing (formation of a permanent mass of cells and secreted macromolecules above the medium level under aeration) is largely reduced as compared to a suspension culture;

6 - Contrary to the entrapment techniques,

-growth occurs without support matrix disruption,

-no additional barrier to mass transfer is imposed on the process, besides diffusion in the biofilm;

7 - This last point makes this technique particularly attractive for the production of secondary metabolites from fungus-elicited plant cells. It meets the requirements for rapid and direct contact between the plant cells and the macromolecular and/or fibrilar species of the fungal extracts used for elicitation. It makes frequent medium changeovers possible throughout the culture process without biomass leakage (Eilert U., Kurz W.G.W. and Constabel F., in The Proceedings of the VI International Congress of Plant Tissue and Cell Culture, A.R. Liss Inc., N.Y. 1986).; and

8 - As shown in Table 1, the loading capacity of this technique compares advantageously to all other immobilization techniques and even to the difficult suspension culture of plant cells. The closest prior technique is the foam particle approach which is difficult to handle hydrodynamically without biomass attrition.

TABLE 1 - Biomass Loading Capacity of Plant
Cells Culturing Systems

| Culturing Systems | Plant Cells | Biomass Loadings | Reference |
|---|---|---|---|
| Surface Immobilized Plant Cells:<br>- Shake flask<br>  (20 mg. d.w./cm$^2$)<br><br>- Laboratory<br>  bioreactor | Catharanthus roseus<br><br><br>C. roseus | 34.8 g d.w./L of wet biomass plus matrix<br><br>37.5 g d.w./L of medium<br>32.0 g d.w./L of wet biomass plus matrix<br>or equivalent to 16.0 g d.w./L of suspension | this work<br><br><br>this work |
| Calcium alginate entrapment | C. roseus | 12.0 g d.w./kg of beads | Morris P., Smart N.J., & Fowler M.W., Plant Cells Tissue Organ Culture 2, 207 1983 |
| Hollow fiber cartridge | Daucus carota | 0.52 g d.w./L of medium | Jose W., Pedersen H. & Chen C., Annals of the N.Y. Academy of Sciences 413, 409, 1983 |

## TABLE 1 Continued

| Polyurethane foam particles in shake flask | _Capsicum_ _frutescens_ | 32.4 g d.w./L of empty foam particles | Lindsey K., Yeoman M.M., Black G.M. & Mavituna F., FEBS Letters 155, 1, 143, 1983 Mavituna F. & Park J.M., Biotech. Letters 7, 9, 637, 1985 |
|---|---|---|---|
| Suspension Culturing<br><br>- Shake flask | _C. roseus_ | 32.5 g d.w./L of suspension | Fowler M.W., Biotech. and Genetic Engineering Reviews, 2, 41, 1984 |

TABLE 1 Continued

| - Airlift | C. roseus | 14.0-20.0 g d.w./ L, of suspension | Fowler M.W. op. cit. Fowler M.W. in Plant Biotech. Mantel S.H. & Smith H. ed., Cambridge Un. Press, Cambridge U.K. 1983, p. 3 Smart N.J. & Fowler M.W., J. of Experimental Botany 35, 531, 1984 Smart N.J. & Fowler M.W. Applied Biochemistry & Biotechnology 9, 209, 1984 |
| - Mechanically mixed fermenter (modified impeller) | Cudrania tricuspidata | 30.0 g d.w./L of suspension | Tanaka H., Biotech. & Bioengineering 23, 1203, 1981 |

This development addresses the selection of suitable support materials to immobilize plant cells on the surface of the support, the mechanism of the surface adhesion process, the loading characteristics of surface immobilized plant cells (SIPC) cultured in flasks, and the development of laboratory size bioreactors for the culturing and immobilization of plant cells.

In accordance with this invention, a method of immobilizing plant cells onto a support material is provided comprising:

a) providing a support material in the form of a multiplicity of fibres loosely bound together, said fibres being selected from polyester fibres and cellulosic fibres;

b) configuring the selected material in a suitable culture vessel;

c) providing that plant cell growth medium is present in said vessel and that the vessel contents are sterile;

d) inoculating the sterile medium of step c) with a suitable suspension of plant cells;

e) circulating the plant cells for sufficient time and under conditions selected to allow the cells to become immobilized on the fibrous support material; and

f) growing the immobilized cells for sufficient time and under selected conditions to reach a suitable biomass concentration.

In the preferred embodiments of the present invention the plant cells to be immobilized are selected from the group consisting of: <u>Catharanthus</u> <u>roseus</u> and <u>Papaver</u> <u>somniferum</u>.

In the preferred embodiment of the present invention the fibrous support material is a fibrous polyster geotextile with a minimum thickness of about 0.5 mm and it is formed into a vertical spiral configuration.

According to the present invention the plant cells to be immobilized are from a growing or stationary suspension and are inoculated at a ratio of at least 5% (v/v) and are circulated so as to contact the fibres for about 1-2 days.

Further, according to the present invention the immobilized plant cells are allowed to grow for about 6-14 days to reach a suitable biomass concentration.

In some embodiments of the present invention the plant cells are grown under mechanical agitation from about 100 to 600 stirrer rpm and sterile air is provided to the medium at a rate of from 0.01 to 0.20 vvm.

There is further provided by this invention the combination of fibrous support material selected from polyester geotextile-type materials and cellulosic fibres loosely bound together by adhesive resins, with plant cells, selected from the group consisting of: <u>C</u>. <u>roseus</u> and <u>P</u>. <u>somniferum</u>, immobilized thereon.

The present invention therefore provides an efficient culture system for the production of secondary metabolites from surface immobilized plant cells. In particular, it provides for: maximum productivity of the metabolic conversion products; efficient harvesting of the products; controlled, well-adapted and uniform culture environment; efficient support material, biomass and medium interaction and stability of the immobilized biomass, allowing for re-use biomass.

The bioreactor and matrix configuration are designed to fulfil the needs of the cultured fragile plant cells and of the production process. The factors that affect this design are:

1 - The functions of the matrix structure are to provide an immobilizing surface area for plant cells enabling desirable, hydrodynamics of the bioreactor system at full wet biomass loading, cell/medium close and rapid contact, efficient and uniform initial biomass deposition and effective retention of the viable immobilized plant cells;

2 - The functions of the basic hydrodynamics of the system are liquid flow uniform distribution of the initially suspended plant cell fragile biomass inoculum throughout the solid matrix arrangement and low shear, efficient, uniform and controllable pumping and mixing of the medium within the matrix structure; and

3 - The functions that the system must perform for the process are: aeration of the culture for survival of the biomass; strict sterile containment for long term operation; high reliability for long term operation and frequent complete medium changes.

## 1 - MATRIX STRUCTURE

A number of different types of solid support materials have been tested for toxicity, growth and attachment of plant cells. The primary requirement for cell attachment appears to be both a highly porous and fibrous surfaces. Also it would seem that the basic chemical nature of the immobilizing material, within the range of the evaluated products, is of lesser importance. However, the chemical composition of these materials could play some role in the mechanism of attachment and/or in the production of secondary metabolites or conversion products.

Most non-metallic supports were not toxic and allowed growth. It was observed that the plant cells attached to four types of materials: a) a rigid and porous matrix made of cellulosic fibres loosly bound by adhesive resins, for example phenolic or melamine resins; b) a porous ceramic material; c) glass fibre and d) fibrous polyester geotextile materials.

The preferred material was found to be the fibrous polyester geotextile type with a minimum thickness of 0.5 mm to provide for ease of formability. These materials provided for: the best growth and loading rate, sterilizability in the medium without affecting the metabolism of the plant cells, and easier formability - as compared to the other materials tested.

The immobilization efficiency of this technique, i.e. the amount of biomass retained over total produced biomass in the culture volume, was relatively low (5 to 65%) in shake flasks. This efficiency was largely improved up to 80 to 99% when using the flask magnetically stirred culturing system, employing a vertical spiral configuration of the solid support.

The superior immobilization efficiency of the technique was later confirmed upon scale-up to the laboratory bioreactors. Almost complete immobilization (≥95%) was consistently observed in these larger vessels. The medium was totally free of suspended plant cells within 2 days, for the magnetic stirred system or within 5 minutes to 17 hours for the bioreactors. Some non-immobilized biomass accumulated mainly in the medium foam layer. The main factors that improved this efficiency and the rate of initial attachment were:

the use of polyester geotextiles 7612 and 7607 for surface immobilization; the availability of a larger immobilizing area; and

the matrix configuration (vertical spiral wound matrix) and the initial suspension mixing pattern, (mechanical stirring).

Although the vertical spiral wound configuration was found to be preferred other configurations are operable.

Following the immobilization of the inoculum, the plant cells grow on both sides of the solid support matrix to attain a suitable biomass concentration. A biomass loading of up to about 20 mg d.w. per $cm^2$ of the textile surface has been attained. Higher levels of biomass loading are possible however a compromise must be reached between biomass loading and mass transfer.

This high biomass retention efficiency was attained under normal culturing conditions using the standard growth medium without special cell attachment promoting additives.

The absence of light does not affect the immobilization process. The inoculum age, and not necessarily its volume, is a determining factor in maximizing loading of surface-immobilized plant cells, at least in shake flask cultivation. The best results were obtained using growing on stationary cells. This result can be associated with the stickiness of older cells, which is involved in the immobilization. The inoculum age will be dependent on the plant cell culture being used, as not all types of plant cells will grow at a similar rate.

The plant cells can be inoculated at a volume: volume ratio of at least 5%, however higher inoculation rates can be used.

The ratio of matrix support (surface area) to culture medium (volume) used in the following examples ranges from about 0.3 $cm^{-1}$ to about 1.2 $cm^{-1}$. However, it has been determined that the higher A/V ratio the better, but a compromise between the immobilizing area and crowding within the bioreactor must be made. Also, the spacing between 2 layers of immobilizing material must be about 1 cm.

## 2 - BASIC HYDRODYNAMICS

The basic hydrodynamics of the bioreactor system must provide for the desirable uniform pumping, distribution and mixing of the fluid throughout the support matrix. It must ensure sufficient agitation of the liquid phase for good mass transfer to and from the immobilized biomass at low shear. The flow pattern must permeate uniformly the whole loaded matrix without creating dead volumes, bridging or channelling and allow rapid liquid circulation and contact with the biomass, especially when using the product elicitation technology.

In this context the fixed vertical spiral wound matrix configuration installed in a suitable bioreactor for example either an airlift bioreactor or in a mechanically stirred bioreactor appears to be an effective system. In addition to fulfilling the basic requirements of the biomass, immobilization technique and process, it provides for: effective long term sterile containment, high reliability and flexibility of operation and a good scale up potential.

## 3 - FUNCTIONS THAT THE SYSTEM MUST PERFORM

Efficient mixing within the matrix structure is necessary to ensure a uniform biofilm growth and to minimize foaming. Suitable mixing can be provided by mechanical stirring or by air flow in a air lift bioreactor. The spiral wound configuration of the solid support has also contributed to the efficient mixing within the matrix structure.

Culture aeration is necessary to supply sufficient $O_2$ throughout the structure yet controlled to avoid foaming or attaining toxic $O_2$ levels. This control can be attained by balancing aeration with mixing in a mechanically agitated reactor, or with air flow control within a given structure of an air lift bioreactor.

This surface immobilization technique has been found effective to immobilize C. roseus plant cells for the production of indole alkaloids (metabolites). It can also be used for any other type of plant cells: for example, P. somniferum plant cell lines have also been surface immobilized successfully. Immobilized P. somniferum cells would be used for the production of secondary metabolites such as: codeine, thebaine and sanguinarine.

The technique can also be applied to other areas such as:

1 - Use of plant cells for biotransformation, (conversion of added substance e.g. for fine chemical production) and for absorption systems, e.g. for metal removal;

2 - Immobilization of plant cells for biosensors; and

3 - Surface immobilization as a physiological study tool.

EXAMPLE 1: THE SELECTION OF SUITABLE SUPPORT MATERIALS TO SURFACE - IMMOBILIZE PLANT CELLS

Flat cut-out samples of materials considered as immobilization supports were sterilized in a 70% ethyl alcohol solution for 30 minutes. They were washed with sterile growth medium 1B5 (B5, Gamborg O.L., Miller R.A. and Ojima K., Exper. Cell Res. 50: 151-159, 1968, containing 2mM of the growth hormone 2,4-dichlorophenoxyacetic acid) and transferred to petri dishes containing 10-20 mL of this growth medium. These were inoculated with 2 mL of a growing culture of either Glycine max, Nicotiana tabacum or C. roseus plant cells. Suspension cultures of these cell lines were obtained from callus. These suspension cultures were grown in 500 mL Delong™ flasks containing 200 mL of liquid medium B5 supplemented with 2 mM of the growth hormone 2,4-dichlorophenoxyacetic acid and 20 g/L of sucrose. The pH value of this medium was adjusted to 5.5 before sterilization (120° C, 15 psi, 1h). The plant cell lines were subcultured (10% (v/v) inoculum) weekly. The cell suspensions were maintained at 27° C under continuous illumination and agitated on a rotary shaker at 150 rpm. Inoculated petri dishes were installed on a rotary shaker operating at 50 rpm and maintained at 27° C for periods of 3 to 7 days.

Many different types of materials were tested for toxicity, growth and attachment of the three cell cultures. They include:

1. Beads of polystyrene, polyethylene, polypropylene and polyvinyldene fluoride.

2. Porous polyethylene sheets.

3. Polyester geotextile-type sheets.

4. Porous matrices made of fibers bound by resins.

5. Polyester-imide surfaces.

6. Negatively and positively charged zeta potential membranes.

7. Coated aluminum sections from honeycomb structures.

8. Hydroculture support fragments (ceramic, oil shale and vermiculite).

9. Particules of silicate and activated carbon.

10. Diatomaceous earth solids of various porosity.

11. Ceramic materials.

12. Glass fiber.

Selection criteria included visual assessment of plant cell culture growth as well as qualitative evaluation of residual biomass attached to the support after a severe water wash.

Most non-metallic supports were not toxic and allowed growth. It was observed that the plant cells attached only to four types of materials tested: a) a rigid and porous matrix made of cellulosic fibres loosely bound together by phenolic or melamine adhesive resins (W8 and Q8 - supplied by AMF Cumo) with a pore size of 50-125 μm; b) the porous ceramic materials; c) the glass fiber; and d) a series of fibrous polyester geotextile materials (7607, 7609, and 7612 - supplied by Telex Inc.) were shown to attach plant cells in a superior way. The geotextile-type material ranged in thickness from 2.6 - 3.5 mm and each had a pore size of 30-80 μm. The fibrous polyester in a geotextile type format was most preferred because it provided: the best growth and loading rate, sterilizability in the medium without affecting the metabolism of the plant cells, and easier formability - as compared to the other materials tested.

The surface immobilized plant cells (SIPC) were fully viable as determined by fluorescein diacetate staining (J.M. Widholm, Stain Technol. 47, 189, 1972). Continued cell viability was found to be very important, for it has been determined that loss in viability results in detachment from the support materials.

Surface immobilized plant cells were fixed with glutaraldehyde, stained with osmium tetroxide, lead citrate and uranyl acetate and embedded in an epoxy resin according to the standard practice for Transmission Electron Microscopy (TEM) observations. Plant cells immobilized on the surface of selected materials were shown by TEM to come in very close contact with the support surface and partly adapt their shape to the microconfiguration of this surface. At the contact area between the cell wall and the support material, observation of an electron dense layer indicated a secretion by the cells of a compound which seems to be acting as a gap filler and/or glueing agent.

## EXAMPLE 2: SURFACE IMMOBILIZATION OF C. ROSEUS PLANT CELLS IN SHAKE FLASKS

The selected support materials (W8, Q8, 7607, 7609 and 7612) were cut into pieces of 8x30 mm. They were suspended in groups of 3 with stainless steel wires in 500 mL Delong™ flasks, sterilized (120° C, 15 psi, 1 h) with or without medium 1B5 (275 mL) which in the latter case was subsequently added. They were inoculated (5-10% v/v) with a growing suspension of plant cells. These flasks were maintained at 27° C under continuous illumination for periods of 2 to 14 days at mixing speeds of 130 or 150 rpm on a rotary shaker.

Immobilized or suspended plant cells were washed with distilled water and dried in an air circulating oven at 60° C for 18 to 24 h to assess the biomass loading of the support materials or the residual dried suspended biomass.

The carbohydrate composition of the culture medium was analyzed with a Waters Scientific HPL Chromatograph Model 6000A-U6K-R401 equipped with a Refractive Index Detector. the separating column used was a Biorad Aminex® HPX-87C. HPLC grade water was the liquid phase. The column was operated at 80° C and at a flow rate of 0.9 mL/min. with a back pressure of 800 psig.

The surface immobilization of growing plant cell suspensions cultured in shake flasks resulted in relatively uniform biomass loading of support materials tested.

Various parameters were evaluated to maximize the biomass loading. Results are presented in Table 2. Loading of plant cells on the support surface depended mainly on the immobilizing material, on the inoculum age and on the mixing speed. The combination of gentle mixing and 7607, 7609 and 7612 materials, which differed only in their thickness, (7607-2.6 mm; 7609-3.0 mm; 7612-3.5 mm) resulted in (200-400%) higher biomass loadings than those observed with materials W8 and Q8. The geotextile materials can also be sterilized in the medium without inhibiting the subsequent growth of plant cells.

The inoculum age was found to be a factor in increasing biomass loading. Inocula from a 8 to 14 day old culture gave the best results. This can be ascribed to the higher secreted polysaccharide content of older plant cell suspensions and to their "stickiness." Increasing the mixing speed from 130 to 150 RPM improved marginally (20%) biomass loading on the Q8 material while reducing it substantially on the 7607, 7609 and 7612 materials.

During the experiments, it was observed that the surface immobilization of plant cells on free floating supports is not efficient. Light did not affect the surface adhesion of cells. However, the SIPC culture growth must have encountered some kind of restriction because an incomplete carbohydrate consumption was observed at biomass loadings higher than 3 mg d.w./cm$^2$ when the mixing speed was 130 RPM. The plant cell suspensions cultured under identical conditions resulted in complete carbohydrate consumption at the twelfth day of culture ($\mu$~0.24 d$^{-1}$).

The highest loading attained was 20.7 mg d.w. per cm$^2$ of material 7612. The thickness of this biomass layer was of the order of 5 mm, which gives a wet-to-dry biomass ratio of 25 consistent with the 95% hydration level of plant cells.

The efficiency of biomass deposition on the support material reached 25 to 50% in a shake flask system where the immobilizing area was 33 cm$^2$ relative to a suspension volume of 300 mL (A/V (area/volume) ~0.11 cm$^{-1}$). The large quantity of plant cells produced and not attached to the support have masked some other characteristics of the flask SIPC, for example in distinguishing between the adhesion and growth processes.

## TABLE 2

SURFACE IMMOBILIZATION OF C. ROSEUS PLANT CELLS IN SHAKE FLASKS

| Material | Material Sterilized With Medium | Inoculum | | Culture Time (d) | Shaker Speed (RPM) | Suspended Biomass (g d.w./L) | Residual Carbo-hydrates (g/L) | Final pH | Biomass Yield | % Of Biomass Attached | Biomass[1] Loading (mg d.w. /cm$^2$) |
|---|---|---|---|---|---|---|---|---|---|---|---|
| | | % Of Medium | Age (d) | | | | | | | | |
| W8 | No | 9% | 6 | 15 | 130 | 8.4 | 0 | 5.5 | 38.1% | 3.6% | 2.7 |
| Q8 | No | 10% | 8 | 12 | 130 | 3.8 | 11.0 | 5.7 | 46.8% | 26.0% | 10.6 |
| Q8 | No | 4% | 10 | 16 | 130 | 8.7 | 3.9 | 5.3 | 64.0% | 13.1% | 6.0 |
| Q8 | No | 7% | 13 | 14 | 130 | 5.8 | 6.1 | 5.8 | 55.5% | 25.1% | 10.5 |
| Q8 | No | 7% | 14 | 14 | 150 | 2.0 | 12.8 | 5.5 | 55.3% | 50.9% | 12.6 |
| A07 | Yes | 7% | 6 | 13 | 130 | 6.8 | 6.6 | 5.5 | 54.5% | 9.4% | 8.0 |
| A07 | Yes | 7% | 6 | 14 | 150 | 7.5 | 0 | 5.7 | 38.9% | 7.1% | 6.0 |
| A07 | Yes | 7% | 12 | 14 | 150 | 8.3 | 0 | 5.3 | 45.7% | 7.9% | 6.1 |
| A07[2] | Yes | 4% | 13 | 14 | 150 | 8.1 | 0 | 5.4 | 42.0% | 1.7% | 1.6 |
| A07 | No | 4% | 12 | 14 | 150 | 7.9 | 0 | 5.4 | 43.4% | 7.9% | 5.7 |
| A09 | Yes | 7% | 6 | 13 | 130 | 3.5 | 10.9 | 5.2 | 48.7% | 19.6% | 9.0 |
| A09 | Yes | 7% | 6 | 14 | 150 | 8.2 | 0 | 5.7 | 43.2% | 7.9% | 5.7 |

TABLE 2 (CONT'D.)

SURFACE IMMOBILIZATION OF C. ROSEUS PLANT CELLS IN SHAKE FLASKS

| Material | Material Sterilized With Medium | Inoculum | | Culture Time (d) | Shaker Speed (RPM) | Suspended Biomass (g d.w./L) | Residual Carbo-hydrates (g/L) | Final pH | Biomass Yield | % Of Biomass Attached | Biomass Loading [1] (mg d.w. /cm$^2$ |
|---|---|---|---|---|---|---|---|---|---|---|---|
| | | % Of Medium | Age (d) | | | | | | | | |
| A12 | Yes | 7% | 6 | 13 | 130 | 6.8 | 5.7 | 5.6 | 56.0% | 22.2% | 9.5 |
| A12 | Yes | 7% | 6 | 14 | 150 | 8.4 | 0 | 5.7 | 45.7% | 9.5% | 6.6 |
| A12 | Yes | 4% | 10 | 14 | 130 | 4.9 | 8.3 | 5.3 | 58.6% | 29.9% | 16.3 |
| A12 | Yes | 4% | 10 | 14 | 130 | 4.9 | 7.7 | 5.3 | 63.2% | 35.6% | 20.7 |
| A12[3] | Yes | 4% | 10 | 14 | 130 | 5.1 | 6.9 | 5.4 | 51.1% | 29.8% | 17.0 |
| A12 | Yes | 7% | 14 | 14 | 150 | 7.7 | 0 | 5.6 | 41.9% | 7.3% | 4.7 |
| A12 | Yes | 7% | 14 | 14 | 150 | 7.4 | 0 | 5.5 | 40.6% | 8.2% | 5.6 |

(1) Silk dried biomass loading mg d.w./cm$^2$ of support area.
(2) Free floating support.
(3) Culture without illumination.

0 291 232

The biomass loading of SIPC cultured in shake flasks was evaluated by periodic retrieval of flask and assessing the amount of immobilized biomass. Results are presented in Table 3 for materials 7612 and Q8 at two mixing speeds. The amount of immobilized plant cell biomass in the shake flask system showed a significant linear correlation with time as long as nutrients are not depleted. The effect of the agitation intensity on the growth rate of immobilized cells for each material was mentioned previously. Increasing the mixing speed lowered the SIPC growth rate on material 7612 to 74% of original value. The same increased mixing improved significantly (100%) the SIPC growth rate on material Q8. The growth rates were similar to the results presented in Table 4 for the magnetic stirrer system (discussed in Example 3).

## EXAMPLE 3: SURFACE IMMOBILIZATION OF C. ROSEUS PLANT CELLS ON MATERIAL 7612 IN THE MAGNETIC STIRRER SYSTEM

Strips of support material (7612) were formed into a vertical spiral configuration of 4.0 cm outside diameter with 100 cm² total surface area. The wound support bundle was suspended in a widemouth Erlenmeyer flask (500 mL) containing 300 mL of medium 1B5. This culture vessel was sterilized, inoculated and maintained at 27°C as in Example 2. Agitation was by a magnetic bar (1cmx5cm) at 110 to 600 RPM. In certain cases, sterile air was passed through a submerged sintered glass sparger at a rate of 0.02 vvm (volume of air/volume of medium/min.). During the first day of culture, low mixing (110-125 rpm) was provided for efficient plant cell attachment. Subsequently, the mixing rate was increased to the desired levels. Alternatively, the mixing can remain constant at about 200 rpm.

Much higher attachment efficiencies (80-90%) were attained in the SIPC culturing system employing magnetic bar agitation in flasks with a A/V ratio (area/volume) of ~0.3 cm$^{-1}$. Complete immobilization of the inoculum occurred generally within the first or second day of culture. The medium was completely free of cells until harvesting, with the little free biomass produced being either attached to the flask or accumulating in the small foam layer in the air-sparged system.

Results of culture experiments in this system are presented in Table 4. High loadings and uniform cell coverage of the support material were observed. Final medium pH was lower than in normal plant cell suspension cultures (pH~5.6-5.8) and SIPC cultured in shake flasks. Restricted growth and altered metabolism of the SIPC were indicated by the low growth rates and biomass yields as well as by incomplete carbohydrate consumption, as compared to suspension cultures ($\mu$~0.24 d$^{-1}$ and yield ~50-60%). In fact this lower yield and the requirement for forced aeration for continued growth suggest higher respiration rates of the SIPC than for suspension cultured cells.

An agitation rate of 125 to 200 rpm (Impeller Reynolds Number ~5200-8300 or a tip speed ~33-52 cm/sec) seems to represent a threshold mixing level at which mass transfer is sufficient to ensure maximum growth rate. Plant cells remained firmly attached to the support material even at a mixing speed of 600 rpm (Impeller Reynolds Number of 25000 or tip speed of 157 cm/sec).

As in Example 2, the growth rates of SIPC were determined for the magnetic stirrer system. The same linear growth behavior was observed (Table 3) as long as nutrients were abundant. Growth rate values were comparable to those observed in shake flasks (Table 3).

## EXAMPLE 4: MECHANICALLY AGITATED BIOREACTOR (IP)

The mechancially agitated bioreactor used for testing was a 2 L glass vessel with a conical profile bottom. Aeration was provided by a sintered glass sparger and agitation by a magnetic bar. Its main characteristics are summarized in Table 5.

TABLE 3

CORRELATED GROWTH RATE OF C. ROSEUS SIPC

| Culture Method | Material | Mixing Speed (RPM) | Data | Linear Correlation | Correlating Coefficient | Specific Growth rate $(d^{-1})$ |
|---|---|---|---|---|---|---|
| Shakeflask | A12 | 130 | Figure 5 Line 1 | SIPC=0.54t+4.3 | 0.88 | 0.07 |
| Shakeflask | A12 | 150 | Figure 5 Line 2 | SIPC=0.40t+4.0 | 0.84 | 0.06 |
| Shakeflask | Q8 | 130 | Figure 6 Line 1 | SIPC=0.26t+2.2 | 0.74 | 0.11 |
| Shakeflask | Q8 | 150 | Figure 6 Line 2 | SIPC=0.57t+1.9 | 0.96 | 0.10 |
| Magnetic stirrer | A07 | 200 | Figure 7 | SIPC=0.66t+0.7 | 0.96 | 0.14 |

SIPC: mg d.w. of biomass per $cm^2$ of immobilizing surface.

t: time of culture (day).

0 291 232

TABLE 4

**SURFACE IMMOBILIZATION OF C. ROSEUS PLANT CELLS ON MATERIAL A12 IN THE MAGNETIC STIRRER SYSTEM**

| Inoculum | | | Mixing Speed (RPM) | Culture Time (d) | Residual Carbo-hydrates (g/L) | Final pH | Biomass Yield | % Of Biomass Attached | Loading (mg d.w. /cm$^2$ | Average Growth rate | |
|---|---|---|---|---|---|---|---|---|---|---|---|
| % Of Medium | Age (d) | Aeration | | | | | | | | ($d^{-1}$) | ($\dfrac{mg\ d.w.}{cm^2 \cdot d}$)[1] |
| 10% | 9 | Periodic[1] | 110 | 13 | 16.2 | 4.8 | 22.5% | 99.5% | 6.8 | 0.05 | 0.25 |
| 7% | 10 | Sparging | 125 | 14 | 10.6 | 5.1 | 30.6% | 99.5% | 7.8 | 0.08 | 0.38 |
| 10% | 10 | Periodic | 125 | 14 | 14.3 | 4.8 | 18.9% | 97.2% | 7.1 | 0.06 | 0.29 |
| 10% | 10 | Sparging | 125 | 14 | 10.6 | 5.0 | 24.3% | 91.8% | 9.7 | 0.08 | 0.48 |
| 10% | 10 | Periodic | 125 | 14 | 15.0 | 5.1 | 24.9% | 96.6% | 6.8 | 0.07 | 0.29 |
| 10% | 10 | Sparging | 200 | 14 | 8.8 | 5.4 | 29.5% | 78.8% | 9.8 | 0.09 | 0.51 |
| 10% | 10 | Sparging | 300 | 14 | 8.1 | 5.4 | 28.2% | 88.6% | 10.6 | 0.10 | 0.57 |
| 10% | 10 | Sparging | 400 | 14 | 7.4 | 5.4 | 29.2% | 89.4% | 12.1 | 0.10 | 0.67 |
| 10% | 10 | Sparging | 500 | 14 | 8.1 | 5.3 | 27.5% | 90.1% | 10.7 | 0.10 | 0.58 |
| 10% | 10 | Sparging | 600 | 14 | 6.9 | 5.0 | 26.8% | 96.3% | 9.3 | 0.11 | 0.52 |

(1)   Average net growth rate
(2)   Flask opening every 2 days

0 291 232

## TABLE 5

### CHARACTERISTICS OF BIOREACTOR IP

| Characteristics | Dimension |
|---|---|
| Height | 20.0 cm |
| Inside diameter | 13.0 cm |
| Stirring bar - length | 7.7 cm |
|        - diameter | 1.2 cm |
| Operational liquid volume (V ) | 1.93 L |
|        - height | 14.9 cm |
| Immobilizing Structure | |
| Support matrix - width | 10.0 cm |
|        - length | 85.0 cm |
|        - Area (A) | 1728.0 cm$^2$ |
| Layers spacing at zero loading | 1.35 cm |
| A/V | 0.89 cm$^{-1}$ |

The solid support was formed into a spiral configuration as described in Example 3. The bioreactor was sterilized with medium according to the standard practice (121°C, 15psi, 1 hr). It was then inoculated (10% v/v) with 8-9 day old flask cultured suspension of C. roseus cells. A mixing rate of 300 rpm (Impeller Reynolds Number of 29600 or a tip speed of 121 cm/sec) and an aeration rate of 0.2 vvm were found optimum for this bioreactor. As observed in the flask systems, complete plant cell immobilization occurred within 24 to 48 hours from inoculation. Uniform biomass coverage of the matrix surface was attained resulting from the swirling motion provided by the mixing bar to the culture broth within the low height-to-diameter (0.77) spiral structure of the supporting matrix. Foaming and frothing in the IP bioreactor was limited to a small (≤ 1 cm) layer once most cells were readily and strongly attached to the immobilizing matrix.

Results from two typical experiments are shown in Table 6.

### TABLE 6

### EXPERIMENTAL TESTING OF MECHANICALLY AGITATED BIOREACTORS

| Parameter | Units | IP-1 | IP-2 |
|-----------|-------|------|------|
| Immobilization Area (A) | cm$^2$ | 1658.0 | 1790.0 |
| Liquid Volume (V) | L. | 1.96 | 1.96 |
| Temperature (T) | °C | 28.0 | 27.0 |
| Culture time (t) | d | 3 | 7 |
| Final culture pH | pH units | 5.2 | 5.5 |
| Final carbohydrates | g/L | 16.2 | 4.6 |
| Average carbohydrate consumption rate | g/L.d | 1.59 | 2.45 |
| Surface immobilized plant cells | mg/cm$^2$ | 5.4 | 8.8 |
| Immobilized biomass fraction | % | 90.9% | 94.7% |
| Biomass yield | % | 75.0% | 52.6% |
| Avg. specific growth rate | d$^{-1}$ | 0.58 | 0.33 |
| Net avg. biofilm growth rate | mg/cm$^2$.d | 1.48 | 1.13 |
| Equivalent suspension biomass concentration | g d.w. L | 4.7 | 9.7 |

These results show that high plant cell retention (≥90%) was observed.

### EXAMPLE 5: SECONDARY METABOLITES FROM IMMOBILIZED PLANT CELLS

Following the immobilization and growth of C. roseus cell line 953 on the surface of the solid support the cell-coated supports were transfered and maintained for three weeks in the alkaloid-producing medium (APM) (Zenk M.H., El-Shagi H., Arens, H., Stöckigt, J., Weiler, E.W., and Deus, B., in Plant Tissue Culture - Biotechnological Application Ed Barz, W., Reinhard, E., Zenk, M.H., p. 27-43 1975, Springer-Verlag New York). A preliminary evaluation revealed that tryptamine was present in the immobilized cells and tryptamine and ajmalicine were present in the medium. Therefore, these results demonstrate that the presence of the solid support did not affect the production of secondary metabolites from the immobilized plant cells.

## Claims

1. A method of immobilizing plant cells on the surface of a support material and establishing thereon a viable culture of said cells, characterised in that the plant cells are immobilized onto, and subsequently grown on, a preformed fibrous support material comprising a multiplicity of loosely bound polyester and/or cellulosic fibres by the steps of
a) placing the support material in a sterile culture vessel containing a sterile culture medium for the plant cells;
b) inoculating the sterile culture medium with a suspension of the plant cells to be immobilised and grown on said support material;
c) circulating the sterile culture medium containing said plant cells through the fibrous support material thereby to immobilize the plant cells onto the fibrous support, and
d) allowing the immobilized plant cells to grow on the fibrous support.
2. A method according to claim 1, characterised in that there are used plant cells of the species Catharanthus roseus or Papaver somniferum.
3. A method according to claim 1 or 2, characterised in that the fibres of the support material are loosely abound together as a textile arrangement or by means of an adhesive.
4. A method according to any one of claims 1 to 3, characterised in that the support material has a thickness of at least about 0.5 mm.
5. A method according to any one of claims 1 to 4, characterised in that the fibrous support material is a fibrous polyester geotextile material.
6. A method according to any one of claims 1 to 5, characterised in that the fibrous support material has a spiral configuration.
7. A method according to any one of claims 1 to 6, characterised in that the support has an immobilizing area relative to the volume of the culture medium in the range 0.3 to 1.2 cm$^{-1}$.
8. A method according to any one of claims 1 to 7 characterised in that the cell suspension used in step b) is a growing or stationary suspension.
9. A method according to any one of claims 1 to 8, characterised in that in step b) the plant cells are inoculated into the culture medium at a ratio of at least about 5% (v/v).
10. A method according to any one of claims 1 to 9, characterised in that the culture medium containing the plant cells is circulated in contact with the fibrous support material for from 1-2 days to allow the cells to become immobilized thereon.
11. A method according to any one of claims 1 to 10 characterised in that in step d) the plant cells are grown on the support material for 6-14 days to reach a viable biomass concentration.
12. A method according to any one if claims 1 to 11 characterised in that the culture medium containing the plant cells is maintained in contact with the support material under mechanical stirring at about 100 to 600 rpm and sterile air is provided to the culture medium at a rate from about 0.01 to 0.20 vvm.
13. A method according to claim 12, characterised in that the culture medium containing the plant cells is maintained under mechanical stirring at 200 rpm and (Impeller-Reynolds Number of about 8300 or a tip speed of about 52 cm/sec) and sterile air is provided to the culture medium at a rate of about 0.02 vvm.
14. A method according to claim 12, characterised in that the culture medium containing the plant cells is maintained under mechanical stirring at about 300 rpm and (Impeller-Reynolds Number of about 29600 or a tip speed of 121 cm/sec) sterile air is provided to the culture medium at a rate of about 0.2 vvm.
15. A viable immobilized plant cell mass characterised in that the plant cells are immobilized on a fibrous support material comprising a loosely bound multiplicity of cellulosic and/or polyester fibres.
16. A viable plant cell mass according to claim 15, characterised in that immobilized plant cells are Catharanthus roseus or Papaver somniferum.
17. A viable plant cell mass according to claim 15 or 16, characterised in that the support member is a cellulosic or polyester geotextile.
18. A viable plant cell mass according to claim 17, characterised in that the biomass loading on the support is in the range of 1-20 mg d.w. per cm$^2$ of textile surface.
19. A bioreactor comprising a viable plant cell mass as claimed in any one of claims 15 to 18 in a culture vessel.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | GB - A - 2 180 554 (UNITED KINGDOM ATOMIC ENERGY AUTHORITY) <br> * Claims 1-4,10 * <br> -- | 1,2,15-17,19 | C 12 N 11/08 <br> C 12 N 11/12 <br> C 12 N 5/00 <br> C 12 N 5/02 |
| X | WO - A1 - 86/05 811 (VERAX CORPORATION) <br> * Claims 1-3,31; page 5, lines 17-22; claim 14 * <br> -- | 1,19 | C 12 M 1/40 <br> C 12 M 3/00 |
| A | EP - A1 - 0 197 784 (GENEX CORPORATION) <br> * Claims 1,3,17 * <br> ---- | 1,15 | |

**TECHNICAL FIELDS SEARCHED (Int. Cl 4)**

C 12 N

C 12 M

The present search report has been drawn up for all claims

| Place of search | Date of completion of the search | Examiner |
|---|---|---|
| VIENNA | 11-08-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82